# EUROPEAN PATENT APPLICATION

(11) **EP 4 801 236 A1**
(43) Date of publication of application: **02.09.2026**
(21) Application number: 24882492.2
(22) Date of filing: 25.10.2024
(51) Int. Cl.: H10K 30/50, C07D 487/22, H10K 30/40, H10K 30/86, H10K 85/10, H10K 85/30, H10K 85/50

(54) **PHOTOELECTRIC CONVERSION ELEMENT AND PHOTOELECTRIC CONVERSION DEVICE**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 28.05.2024 JP 2024086013; 23.10.2024 JP 2024186451
(71) Applicant: Canon Kabushiki Kaisha, Tokyo 146-8501 (JP)
(72) Inventor: SEKIDO, Kunihiko, Tokyo 146-8501 (JP); OHSAWA, Tatsuya, Tokyo 146-8501 (JP); NAKAMURA, Nobuhiro, Tokyo 146-8501 (JP); KATO, Nanami, Tokyo 146-8501 (JP); YOSHIDA, Yu, Tokyo 146-8501 (JP); NISHIDA, Tsutomu, Tokyo 146-8501 (JP)
(74) Representative: TBK
(86) International application number: PCT/JP2024/038106
(87) International publication number: WO 2025/089384

(57) **Abstract**

Provided are a photoelectric conversion element and a photoelectric conversion apparatus each having improved conversion efficiency. The photoelectric conversion element includes first and second electrodes, and a photoelectric conversion layer containing a crystal having a perovskite structure, and is characterized in that: the photoelectric conversion element includes, between the photoelectric conversion layer and the first electrode, a charge-transporting layer containing a crystal of a cyclic conjugated compound in which a plurality of pyrrole rings are bonded by conjugated bonds; in a scanning range of a Bragg angle 20 in an X-ray diffraction spectrum of the charge-transporting layer using a CuKα ray, when a peak with the maximum intensity out of peaks that are present in a range of 5.0 to 8.0° is defined as a peak α and a peak with a maximum intensity out of peaks that are present in a range of 26.0 to 29.0° is defined as a peak β, one of the peak α or the peak β is a peak with the maximum intensity in the scanning range; and when the intensity of the peak α is defined as Iα and the intensity of the peak β is defined as Iβ, a ratio Iα/Iβ is 2.0 or less.

## Description

### [Technical Field]

The present invention relates to a photoelectric conversion element and a photoelectric conversion apparatus.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage by utilizing a photovoltaic effect in which light energy is absorbed from sunlight to generate an electron and a hole.

Currently, an n-p diode-type silicon (Si) single crystal-based solar cell having a light energy conversion efficiency of more than 20% is widely known, and is actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and can be produced in a so-called roll-to-roll system using a sheet-shaped substrate, and hence a cost reduction can be expected. However, further improvements in power generation efficiency and durability have been desired for the practical use of the organic solar cell. In particular, the development of a perovskite solar cell including a crystal having a perovskite structure as a photoelectric conversion layer toward its practical use has been advanced because the cell is excellent in photoelectric conversion property. In, for example, Patent Literature 1, there is a description that conversion efficiency is improved by forming a layer containing a phthalocyanine compound between a hole-transporting layer (hereinafter also referred to as "charge-transporting layer") and a perovskite. In Non Patent Literature 1, there is a description that conversion efficiency is improved by incorporating copper phthalocyanine into a hole-transporting layer.

### [Citation List]

### [Patent Literature]

PTL 1: Japanese Patent Laid-Open No. 2022-168820

### [Non Patent Literature]

NPL 1: F. Wang, et al, J. Phys. Chem. C 2017, 121, 1562

### [Summary of Invention]

### [Technical Problem]

According to an investigation made by the inventors of the present invention, a further improvement in conversion efficiency toward practical use has been required to be achieved in the above-mentioned related art.

The present invention is directed to providing a photoelectric conversion element and a photoelectric conversion apparatus each having improved conversion efficiency.

### [Solution to Problem]

The above-mentioned provision is achieved by the present invention described below. That is, a photoelectric conversion element according to the present invention is a photoelectric conversion element including:
a first electrode; a second electrode; and
a photoelectric conversion layer arranged between the first electrode and the second electrode, the photoelectric conversion layer containing a crystal having a perovskite structure,
wherein the photoelectric conversion element further includes,
between the photoelectric conversion layer and the first electrode, a charge-transporting layer containing a crystal of a cyclic conjugated compound in which a plurality of pyrrole rings are bonded by conjugated bonds,
wherein, in a scanning range of a Bragg angle 2θ of 3.0 to 30.0° in an X-ray diffraction spectrum of the charge-transporting layer using a CuKα ray, when a peak with a maximum intensity out of peaks that are present in a range of 5.0 to 8.0° is defined as a peak α, and a peak with a maximum intensity out of peaks that are present in a range of 26.0 to 29.0° is defined as a peak β, one of the peak α or the peak β is a peak with a maximum intensity in the scanning range, and
wherein, when an intensity of the peak α is defined as Iα and an intensity of the peak β is defined as Iβ, a ratio Iα/Iβ is 2.0 or less.

### [Advantageous Effects of Invention]

According to the present invention, the photoelectric conversion element having improved conversion efficiency can be provided.

### [Brief Description of Drawings]

[Fig. 1]

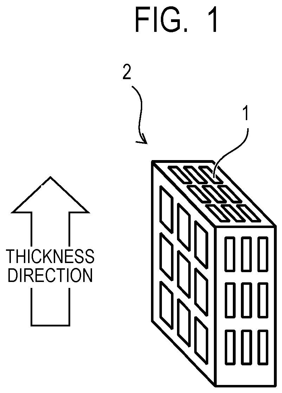

   Fig. 1 is a schematic view of the crystal structure of a cyclic conjugated compound in which a plurality of pyrrole rings are bonded by conjugated bonds, the cyclic conjugated compound being included in a charge-transporting layer according to each of Examples of the present invention.
[Fig. 2]

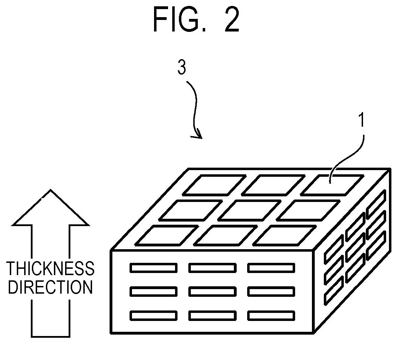

   Fig. 2 is a schematic view of the crystal structure of a cyclic conjugated compound in which a plurality of pyrrole rings are bonded by conjugated bonds, the cyclic conjugated compound being included in a charge-transporting layer according to each of Comparative Examples of the present invention.
[Fig. 3]

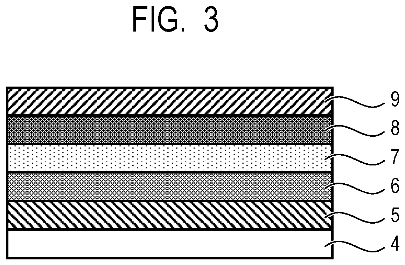

   Fig. 3 is a schematic sectional view in the thickness direction of a photoelectric conversion element according to a first embodiment of the present invention.
[Fig. 4]

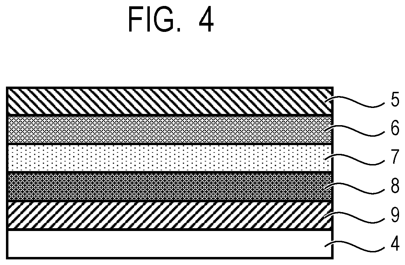

   Fig. 4 is a schematic sectional view in the thickness direction of a photoelectric conversion element according to a second embodiment of the present invention.
[Fig. 5]

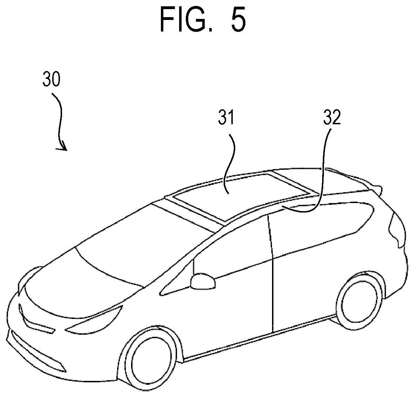

   Fig. 5 is a perspective view for schematically illustrating an embodiment of a moving body including the photoelectric conversion element of the present invention.
[Fig. 6]

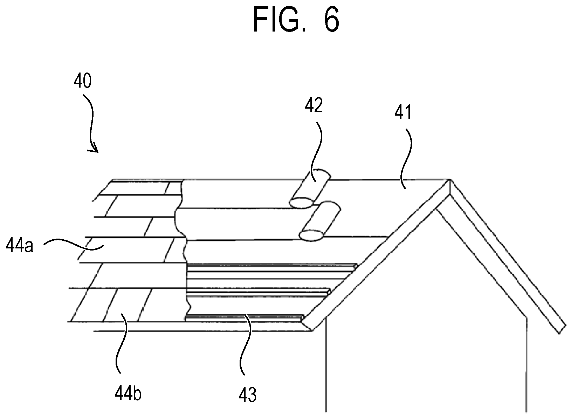

   Fig. 6 is a perspective view for schematically illustrating an embodiment of a building material including the photoelectric conversion element of the present invention.

### [Description of Embodiments]

A photoelectric conversion element of the present invention is a photoelectric conversion element including: a first electrode; a second electrode; and a photoelectric conversion layer arranged between the first electrode and the second electrode, the photoelectric conversion layer containing a crystal having a perovskite structure. The photoelectric conversion element includes, between the photoelectric conversion layer and the first electrode, a charge-transporting layer containing a cyclic conjugated compound in which a plurality of pyrrole rings are covalently bonded.

The inventors of the present invention have found that a photoelectric conversion element with excellent conversion efficiency is provided by including the above-mentioned charge-transporting layer. Details of the reason why high stability of the photoelectric conversion element can be obtained in the present invention are not clear, but the inventors have presumed the mechanism to be as described below.

According to a related-art document, when a crystal having a perovskite structure is included in a photoelectric conversion layer, submicron unevenness occurs on its surface. The inventors of the present invention have estimated that the interfacial bonding of the photoelectric conversion layer with an electrode is stabilized by the filling of a recess of such unevenness with a pigment particle including a phthalocyanine compound, which is a kind of cyclic compound formed of a plurality of pyrrole rings covalently bonded, and hence high photoelectric conversion efficiency can be obtained. However, the inventors have found that the reason alone is insufficient for providing higher conversion efficiency.

In view of the foregoing, the inventors of the present invention have inferred that the formation of a charge-transporting layer containing a cyclic conjugated compound in which a plurality of pyrrole rings are covalently bonded and showing a specific X-ray diffraction peak contributes to the improvement in conversion efficiency.

Specifically, the improvement in conversion efficiency can be achieved under the following conditions: in the scanning range of a Bragg angle 2θ of 3.0 to 30.0° in the X-ray diffraction spectrum of the charge-transporting layer using a CuKα ray (λ=1.5418 Å), when a peak with the maximum intensity out of peaks that are present in a range of 5.0 to 8.0° is defined as a peak α, and a peak with the maximum intensity out of peaks that are present in a range of 26.0 to 29.0° is defined as a peak β, one of the peak α or the peak β is a peak with the maximum intensity in the scanning range; and when the intensity of the peak α is defined as Iα and the intensity of the peak β is defined as Iβ, a ratio Iα/Iβ is 2.0 or less.

The inventors of the present invention have conceived that the strong peak intensity (Iα) in the range of 5.0 to 8.0° indicates that there are many crystals (Fig.
1) of the cyclic conjugated compound stacked in a lateral direction (molecular spacing of about 12 Å assuming from Bragg's equation), and the strong peak intensity (Iβ) in the range of 26.0 to 29.0° indicates that there are many crystals (Fig.
2) thereof stacked in a longitudinal direction (thickness direction) (molecular spacing of about 3 Å assuming from Bragg's equation). That is, a smaller ratio Iα/Iβ indicates a higher ratio of crystals stacked in the longitudinal direction with respect to crystals stacked in the lateral direction.

The inventors of the present invention have inferred that the presence of the crystals stacked in the longitudinal direction at a certain ratio or more contributes to the improvement in conversion efficiency because stacking in the longitudinal direction means stacking in a direction in which π-electron clouds overlap and hence high transportation efficiency of carriers moving within a film is expected.

Fig. 1 is an illustration of an entire crystal 2 containing cyclic conjugated compound molecules 1 (plate-like structures) in each of which a plurality of pyrrole rings are bonded by conjugated bonds.

Fig. 2 is an illustration of an entire crystal 3 containing the cyclic conjugated compound molecules 1 (plate-like structures) in each of which a plurality of pyrrole rings are bonded by conjugated bonds.

The effects of the present invention can be achieved through the above-mentioned mechanism.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of an element to be incorporated gradually changes, or a layer that may form a complicatedly intricate structure together with another layer.

Fig. 3 is a sectional view for schematically illustrating the configuration of a photoelectric conversion element according to one embodiment of the present invention. The photoelectric conversion element of Fig. 3 includes a substrate 4, and a second electrode 5, an electron-transporting layer 6, a photoelectric conversion layer 7, a charge-transporting layer 8, and a first electrode 9 arranged thereon. One of the first electrode 9 and the second electrode 5 is an anode, and the other is a cathode. A current can be extracted by connecting the first electrode 9 and the second electrode 5 with an external circuit.

The photoelectric conversion layer 7 is excited by light, which has entered the layer through the substrate 4, the second electrode 5, and the electron-transporting layer 6, or the first electrode 9 and the charge-transporting layer 8, to generate an electron or a hole. That is, the photoelectric conversion layer 7 generates a current between the first electrode 9 and the second electrode 5. The electron-transporting layer 6 is a layer arranged between the photoelectric conversion layer 7, and the two electrodes 5 and 9, and may not be formed in some cases. A form in which the plurality of electron-transporting layers 6 and photoelectric conversion layers 7 are laminated may be adopted. Such form may also be referred to as "tandem structure."

In addition, a configuration in which the first electrode 9, the charge-transporting layer 8, the photoelectric conversion layer 7, the electron-transporting layer 6, and the second electrode 5 are arranged on the substrate 4 may be adopted (Fig. 4).

The respective members are described below.

### [Photoelectric Conversion Element]

The photoelectric conversion element of the present invention is a photoelectric conversion element including: the first electrode; the second electrode; and the photoelectric conversion layer arranged between the first electrode and the second electrode, the photoelectric conversion layer containing the crystal having a perovskite structure, the element being characterized by further including the charge-transporting layer between the photoelectric conversion layer and the first electrode. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a crystal having a perovskite structure in its photoelectric conversion layer.

A method of forming each of the layers including the photoelectric conversion layer and charge-transporting layer of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The respective layers are described below.

### [Substrate]

The photoelectric conversion element of the present invention may include the substrate, and examples thereof include a transparent glass substrate made of soda-lime glass or alkali-free glass, a ceramic substrate, and a transparent plastic substrate. When light is taken in from the first electrode 9 side in Fig. 4, an opaque material may be used as the substrate 4, and when light is taken in from the second electrode 5 side in Fig. 5, the substrate 4 is formed of a transparent material.

### [Electrode]

A material for the first electrode or the second electrode is not particularly limited, and a material that has hitherto been known may be used. Examples thereof include: metals, such as gold, silver, titanium, and copper; sodium; a sodium-potassium alloy; lithium; magnesium; carbon; a carbon nanotube; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof. At least one electrode of the first electrode or the second electrode on a light incident side is a transparent electrode, and the other may be a transparent electrode or may also serve as a reflective layer formed of a light reflective material, or may be a transparent electrode including a reflective layer on a side opposite to the light incident side. The photoelectric conversion element is preferably such that the first electrode is a positive electrode. When the first electrode is on the light incident side, the second electrode and the substrate may be a transparent electrode and a reflective layer, respectively. The transparent electrode may be a patterned electrode.

### [Photoelectric Conversion Layer]

The photoelectric conversion layer 7 contains the crystal having a perovskite structure. The crystal having a perovskite structure to be used in the present invention is preferably represented by the following general formula [1]. ${ABX}_{3}$

In the general formula [1], A represents a monovalent cation of an organic molecule or a metal atom, B represents a divalent metal cation, and X represents a monovalent halide anion.

A in the general formula [1] preferably represents CₚN_{q}Hᵣ ("p", "q", and "r" each represent a positive integer) in the case of, for example, the organic molecule. Specific examples thereof include methylammonium and formamidinium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer. The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formulae [2] to [4] ("n" represents a positive integer). $R {′}_{2} {A}_{n - 1} {B}_{n} {X}_{3 n + 1}$ $R " {A}_{n - 1} {B}_{n} {X}_{3 n + 1}$ $R {‴}_{2} {A}_{n} {B}_{n} {X}_{3 n + 1}$

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule or a metal that may have a substituent. Specifically, ethylammonium, propylammonium, n-butylammonium, n-hexylammonium, n-octylammonium, 1,6-hexanediammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbitals. Those metal atoms may be used alone or in combination thereof.

X in the general formulae [1] to [4] represents a halogen atom, and examples thereof include chlorine, bromine, iodine, sulfur, and selenium. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence its application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4FPEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I₁₆, (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred. The A site, B site, or X site of each of the general formulae may be adjusted to be deficient or excessive in accordance with purposes, and the combinations of x1 to x5 may be changed in accordance with purposes. Examples of the combinations of x1 to x5 are as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiophenethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

The crystal having a perovskite structure to be used in the present invention is preferably a crystalline semiconductor. The term "crystalline semiconductor" means a semiconductor that enables the measurement of an X-ray scattering intensity distribution to detect a scattering peak. When the crystal having a perovskite structure is the crystalline semiconductor, the mobility of an electron in the crystal having a perovskite structure increases, and the photoelectric conversion efficiency of the photoelectric conversion element is improved.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

### [Charge-transporting Layer]

The charge-transporting layer 8 of the present invention contains a crystal of a cyclic conjugated compound in which a plurality of pyrrole rings are covalently bonded.

A porphyrin compound or a phthalocyanine compound is preferred, and the phthalocyanine compound is more preferred as the cyclic conjugated compound in which a plurality of pyrrole rings are covalently bonded to be used in the present invention from the viewpoint of the spreading of a π-electron cloud serving as a starting point of an interaction. The phthalocyanine compound may include a central element, and examples of the central element include Ga, Cu, Ti, Zn, Si, V, Pb, Pt, Co, Sn, Mg, Fe, Al, and Mn. Of those, a gallium phthalocyanine compound in which a central element is Ga or a titanyl phthalocyanine compound in which a central metal is Ti is preferred. A hydroxygallium phthalocyanine compound is more preferred.

Specific examples of the cyclic conjugated compound in which a plurality of pyrrole rings are covalently bonded in the present invention are described below:
porphyrin derivatives, such as tetraphenylporphyrin, diphenylporphyrin, tetrapyridylporphyrin, copper porphyrin, copper tetraphenylporphyrin, copper octaethylporphyrin, cobalt tetraphenylporphyrin, octaethylporphyrin, chlorophenylporphyrin, methoxyphenylporphyrin, methylphenylporphyrin, zinc porphyrin, magnesium porphyrin, octabutoxyporphyrin, manganese chloroporphyrin, metal-free tetraazaporphyrin, copper tetraazaporphyrin, zinc tetraazaporphyrin, nickel tetraazaporphyrin, titanyl tetraazaporphyrin, tetraphenyl tetraazaporphyrin, and octaphenyl tetraazaporphyrin;
phthalocyanine derivatives, such as hydroxygallium phthalocyanine, chlorogallium phthalocyanine, copper phthalocyanine, zinc phthalocyanine, phthalocyanine, cobalt phthalocyanine, titanyl phthalocyanine, dichlorotin phthalocyanine, magnesium phthalocyanine, tin phthalocyanine, lead phthalocyanine, iron phthalocyanine, vanadyl phthalocyanine, chloroaluminum phthalocyanine, nickel phthalocyanine, dichlorosilicon phthalocyanine, indium chlorophthalocyanine, manganese phthalocyanine, chloroiron phthalocyanine, and platinum phthalocyanine; and
naphthalocyanine derivatives, such as naphthalocyanine, magnesium naphthalocyanine, copper naphthalocyanine, cobalt naphthalocyanine, vanadyl naphthalocyanine, tin naphthalocyanine, and dichlorotin naphthalocyanine.

The charge-transporting layer may contain a resin, and the content of the resin in the charge-transporting layer is preferably 3 to 30 mass%, more preferably 5 to 20 mass% with respect to the content of the cyclic conjugated compound in which a plurality of pyrrole rings are covalently bonded from the viewpoints of a film-forming property and a charge-transporting ability.

In addition, the molecular weight of the resin is preferably 10,000 or more.

Examples of the resin to be preferably used in the present invention include a polyester resin, a polycarbonate resin, a polyvinyl acetal resin, a polyvinyl butyral resin, an acrylic resin, a polyvinyl alcohol resin, a cellulose resin, a polystyrene resin, a polyvinyl acetate resin, and a polyvinyl chloride resin. The glass transition point of the resin is preferably 60 to 95°C from the viewpoint of the film-forming property.

The charge-transporting layer may contain an aromatic ring compound containing a hydroxy group, and the content of the aromatic ring compound containing a hydroxy group in the charge-transporting layer is preferably 1 to 30 mass%, more preferably 5 to 20 mass% with respect to the content of the cyclic conjugated compound in which a plurality of pyrrole rings are covalently bonded from the viewpoints of the film-forming property and the charge-transporting ability.

An example of the aromatic ring compound containing a hydroxy group to be preferably used in the present invention is a calixarene compound.

Moreover, in the scanning range of a Bragg angle 2θ of 3.0 to 30.0° in the X-ray diffraction spectrum of the charge-transporting layer using a CuKα ray, when a peak with the maximum intensity out of peaks that are present in a range of 5.0 to 8.0° is defined as a peak α, and a peak with the maximum intensity out of peaks that are present in a range of 26.0 to 29.0° is defined as a peak β, one of the peak α or the peak β is a peak with the maximum intensity in the scanning range, and when the intensity of the peak α is defined as Iα and the intensity of the peak β is defined as Iβ, a ratio Iα/Iβ is 2.0 or less.

The ratio Iα/Iβ is preferably 1.5 or less, more preferably 1.0 or less.

In addition, a case where the peak α is present in a range of 7.2 to 7.6° and the peak β is present in a range of 28.0 to 28.4°, and the above-mentioned value of the ratio Iα/Iβ is satisfied is more preferred.

Examples of a dispersion method for forming particles from crystals of the cyclic conjugated compound in which a plurality of pyrrole rings are covalently bonded include methods using a paint shaker, a sand mill, a ball mill, and a liquid collision-type high-speed disperser.

Of those, a sand mill forms the crystals into the particles by the rotation of a disc rotating in a mill and a shearing force by a medium such as glass beads serving as a grinding medium. At that time, the peak ratio changes because crystallinity changes depending on dispersion conditions, such as a dispersion time, the amount of beads, and the number of revolutions of the disc. For example, the ratio Iα/Iβ tends to reduce when the dispersion time is extended to such an extent that over-dispersion (e.g., the aggregation of the particles or the formation of fragments) does not occur.

The thickness of the charge-transporting layer is preferably 1 to 1,000 nm, more preferably 5 to 500 nm, particularly preferably 10 to 200 nm.

The measurement of the X-ray diffraction spectrum of the charge-transporting layer used in the present invention and the determination of the cyclic compound or the like incorporated into the layer in which a plurality of pyrrole rings were covalently bonded were performed by the following method after a layer above the charge-transporting layer of the photoelectric conversion element of the present invention was removed with an organic solvent such as chloroform, and then the surface of the charge-transporting layer was exposed.

### [Analysis of Amount of Compound]

The surface of the charge-transporting layer was wiped with a cotton swab or the like having a solvent applied thereto. The wiped-off component was dissolved in deuterated sulfuric acid and subjected to ¹H-NMR measurement (apparatus: AVANCE III 500, manufactured by Bruker Corporation). In addition, the presence of a compound was recognized by performing the mass/structural analysis of the wiped-off component with GPC, MALDI-TOF-MS, IR, and gas chromatography.

In addition, the photoelectric conversion element was cut and fixed to a tilted sample stage, and then the thickness of the charge-transporting layer was determined with a cross-sectional SEM (apparatus: SmartSEM, manufactured by Carl Zeiss Co., Ltd.).

### ·MALDI-TOF-MS analysis

The molecular weight of the compound was determined from an obtained peak top value under the following conditions.

Measuring instrument used: ultrafleXtreme, matrix-assisted laser desorption ionization time-of-flight mass spectrometer (MALDI-TOF MS) manufactured by Bruker Daltonics K.K.
Acceleration voltage: 20 kV
Mode: Reflector
Molecular weight standard: Fullerene C60

### [X-ray Diffraction Measurement]

The X-ray diffraction spectrum of the exposed charge-transporting layer was measured, and the peak intensity ratio (Iα/Iβ) thereof was calculated.
Measuring instrument used: X-ray diffractometer RINT-TTRII manufactured by Rigaku Corporation
X-ray tube: Cu
X-ray wavelength: Kα1
Tube voltage: 50 KV
Tube current: 300 mA
Scanning method: 2θ-θ scan
Scanning speed: 4.0°/min
Sampling interval: 0.02°
Start angle 2θ: 3.0°
Stop angle 2θ: 30.0°
Goniometer: Rotor horizontal goniometer (TTR-2)
Filter: None
Detector: Scintillation counter
Incident monochromator: Used
Slit: Variable slit (Parallel beam method)
Counter monochromator: Not used
Divergence slit: Open
Divergence longitudinal limiting slit: 10.00 mm
Scattering slit: Open
Receiving slit: Open

### [Second Charge-transporting Layer]

In the present invention, the photoelectric conversion element of the present invention may include a second charge-transporting layer between the charge-transporting layer 8 and the first electrode 9 in Fig. 1 from the viewpoint of the compatibility of a film of the charge-transporting layer.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. The compound particularly preferably has an aromatic ring from the viewpoint of the compatibility of a film interface, and Spiro-OMeTAD, PTAA, or a phthalocyanine compound is preferred.

In addition, the second charge-transporting layer may contain a dopant as an additive in order to improve its charge transportation capability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Electron-transporting Layer]

In the photoelectric conversion element of the present invention, the electron-transporting layer 6 may be arranged between the second electrode 5 and the photoelectric conversion layer 7 as illustrated in Fig. 1.

A material for the electron-transporting layer 6 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide.

A preferred lower limit of the thickness of the electron-transporting layer 6 is 1 nm, and a preferred upper limit thereof is 2,000 nm. When such thickness is 1 nm or more, a hole can be sufficiently blocked, and when the thickness is 2,000 nm or less, the electron-transporting layer 6 is less liable to serve as a resistance at the time of electron transportation, and hence the photoelectric conversion efficiency increases. A more preferred lower limit of the thickness is 3 nm, a more preferred upper limit thereof is 1,000 nm, a still more preferred lower limit thereof is 5 nm, and a still more preferred upper limit thereof is 500 nm.

### <Application Examples>

Application examples of the present invention are directed to a photoelectric conversion apparatus, a moving body, and a building material.

### [Photoelectric Conversion Apparatus]

A photoelectric conversion apparatus may be formed by using the plurality of photoelectric conversion elements of the present invention. When the plurality of photoelectric conversion elements are connected, such photoelectric conversion apparatus may also be referred to as "photoelectric conversion cell" or "photoelectric conversion module." In the photoelectric conversion element, elements having different absorption wavelengths may be laminated to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor.

In order to impart a function of, for example, maintaining or increasing the amount of incident light, a surface layer to which water or dirt is hard to adhere, or a function of collecting or guiding light may be added.

### [Moving Body]

Fig. 5 is a perspective view for schematically illustrating one embodiment of a moving body including the photoelectric conversion element of the present invention. A moving body 30 includes a photoelectric conversion element 31 of the present invention and a body 32 including the photoelectric conversion element 31. The photoelectric conversion element 31 is arranged on the position of the body 32 at which ambient light can be received.

When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

Fig. 6 is a perspective view for schematically illustrating one embodiment of a building material including the photoelectric conversion element of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence its photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

In addition to the application examples described above, the following application examples may be given: portable devices, such as a calculator, a sensor, and a small solar panel; wearable devices, such as a glasses-type terminal, a watch-type terminal, and a portable medical device; sheet structures supported by a plurality of frames, such as a tent, a plastic house, and a loading platform of a truck; and structures to be used by being fixed, such as a road surface panel, a floating panel, a building material utilizing the flexibility of a substrate, a wall-type building material, a glass-type building material, and a mega solar panel.

### [Method of producing Photoelectric Conversion Element]

A method of producing the photoelectric conversion element of the present invention includes the steps of: forming a first electrode; forming a second electrode; and forming a photoelectric conversion layer containing a crystal having a perovskite structure between the first electrode and the second electrode.

The respective steps of the production method are described below.

### [Step of forming First Electrode and Step of forming Second Electrode]

The method of producing a photoelectric conversion element of the present invention includes the step of forming the first electrode and the step of forming the second electrode. In the step of forming the first electrode and the step of forming the second electrode, appropriate methods may be selected in accordance with a material of the first electrode and a material of the second electrode, respectively. Examples of such methods include, but are not limited to, a sputtering method, a vacuum vapor deposition method, a vapor phase growth method (CVD method), and a spray pyrolysis deposition method (SPD method). Materials of the first electrode and the second electrode are as described above. When one, or each of both, of the first electrode and the second electrode is a transparent electrode, the thickness of the transparent electrode is preferably 0.03 to 3 µm.

When a solar cell is produced, cutting processing may be performed for circuit formation between steps. Examples of the cutting processing include mechanical patterning and laser patterning.

### [Modularization Step]

An element formed up to the electrode may be sealed. A sealing method is, for example, sealing with a resin or sealing with a film. Examples of a material used for the sealing include silazane, silicone rubber, resins each having a siloxane skeleton, and glass.

In addition, hairline treatment may be applied to the surface of the sealed element from the viewpoint of the suppression of adhesion between elements occurring during winding in a roll-to-roll system.

### [Step of forming Photoelectric Conversion Layer]

The step of forming the photoelectric conversion layer may include a step of applying a liquid containing the material of the photoelectric conversion layer as described above. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method. The method is appropriately selected therefrom in accordance with the characteristics of a photoelectric conversion layer to be produced, such as thickness control and orientation control. Annealing treatment may be performed under reduced pressure or in an inert atmosphere (in a nitrogen or argon atmosphere) in order to remove a solvent or a dispersion medium from the applied liquid containing the material of the photoelectric conversion layer. The temperature of the annealing treatment is preferably 40 to 300°C, more preferably 50 to 150°C. The annealing treatment is preferably performed because materials for forming the respective layers may permeate each other at an interface between laminated layers to increase a contact area, and hence a short-circuit current can be increased.

### [Step of forming Charge-transporting Layer]

As a step of forming a charge-transporting layer, a method of applying a liquid containing the material of the charge-transporting layer as described above is preferred. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method. In addition, examples of the step of forming the charge-transporting layer include the following methods:
a method including arranging a charge-transporting particle on the surface of the photoelectric conversion layer, and then applying and drying a resin solution in which a resin is dissolved; a method including applying the resin solution in which the resin is dissolved onto the surface of the photoelectric conversion layer, then arranging the charge-transporting particle thereon, and then drying the resin solution; and a method including applying a solution, which is obtained by dispersing the charge-transporting particle in the resin solution in which the resin is dissolved, onto the surface of the photoelectric conversion layer, and drying the solution.

### [Examples]

The present invention is described in more detail below by way of Examples and Comparative Examples. The present invention is by no means limited to the following Examples without departing from the gist thereof. In the description of the following Examples, the term "part(s)" is by mass unless otherwise specified.

### (Production Step for Particle 1 containing Crystal of Cyclic Compound in which plurality of Pyrrole Rings are bonded by Conjugated Bonds)

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine particle (crystal) having a water content of 1.0 mass% or less was obtained.

### Step (3)

1 Part of the hydroxygallium phthalocyanine particle was subjected to dispersion treatment for 100 hours with a sand mill (TSG-1/4G-4U, manufactured by Igarashi Machine Production Co., Ltd. (currently AIMEX Co., Ltd.), disc diameter: 70 mm, number of discs: 5) loaded with 5 parts of an N-methylformamide solvent and 5 parts of glass beads while discs were rotated at 300 rpm. After that, the resultant was filtered and dried to provide a particle 1.

### (Production of Resin Solution 1)

1.0 Gram of polyvinyl butyral (product name: BM-2, manufactured by Sekisui Chemical Co., Ltd.) was dissolved in 19 g of 2-propanol by stirring for 24 hours to provide a resin solution 1.

### (Production of Resin Solution 2)

1.0 Gram of polyacrylic acid (molecular weight: 5,000, manufactured by FUJIFILM Wako Pure Chemical Corporation) was dissolved in 19 g of ethanol by stirring for 24 hours to provide a resin solution 2.

### (Example 1)

### [Formation of Electron-transporting Layer]

A glass substrate with ITO was washed, and tin(II) oxide prepared to 3 mass% was applied thereonto by spin coating. After that, the resultant was heated at 150°C for 30 minutes to form an electron-transporting layer as a thin film having a thickness of 15 nm.

### [Formation of Photoelectric Conversion Layer]

22.4 Milligrams of methylammonium bromide, 172 mg of formamidinium iodide, and 576 mg of lead iodide were dissolved in 600 µL of N,N-dimethylformamide and 160 µL of dimethyl sulfoxide, and were stirred for 1 hour (solution 1). Further, 389.72 mg of cesium iodide was dissolved in 1,000 µL of dimethyl sulfoxide, and the solution was stirred for 1 hour (solution 2). After that, 40 µL of the cesium iodide solution (solution 2) was added to the solution 1 to prepare a coating liquid for a photoelectric conversion layer. The coating liquid was applied onto the electron-transporting layer by spin coating to form a photoelectric conversion layer formed of Cs_{0.05}(FA_{0.83}MA_{0.17})_{0.96}Pb(I_{0.95}Br_{0.05})₃ and having a thickness of 400 nm.

### [Formation of Charge-transporting Layer]

0.1 Gram of the particle 1 and 0.01 g of a calixarene compound (Exemplary Compound 1 described in Japanese Patent Laid-Open No. 2003-207913) were encapsulated in a container with 10.6 g of 2-propanol and 11 g of zirconia beads, and the resultant was subjected to dispersion with a paint shaker (manufactured by Toyo Seiki Seisaku-sho, Ltd.) for 8 hours to prepare a coating liquid for a charge-transporting layer. The coating liquid for a charge-transporting layer was applied onto the photoelectric conversion layer by spin-coating to form a charge-transporting layer having a thickness of 150 nm.

### [Introduction of Second Charge-transporting Layer]

0.15 Gram of Spiro-OMeTAD serving as a material for a second charge-transporting layer was dissolved in 2.2 g of chlorobenzene. 36 Microliters of an acetonitrile solution obtained by dissolving 0.2 g of lithium bis(trifluoromethanesulfonyl)imide in 0.3 g of acetonitrile and 60 µL of t-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed thereinto to prepare a coating liquid for a second charge-transporting layer. The coating liquid was applied onto the above-mentioned charge-transporting layer by a spin coating method to form a second charge-transporting layer having a thickness of 150 nm.

### [Formation of First Electrode]

A gold electrode having a thickness of 80 nm and an area of 0.09 cm² was formed on the second charge-transporting layer by a vacuum vapor deposition method. Thus, a photoelectric conversion element was obtained.

### [Photoelectric Conversion Efficiency Evaluation]

A power source (manufactured by Keithley Instruments, Model 236) was connected between the electrodes of the photoelectric conversion element, and constant light was applied with a solar simulator (manufactured by Yamashita Denso Corporation) at an intensity of 100 mW/cm², followed by the measurement of a current and a voltage to be generated. Thus, photoelectric conversion efficiency was evaluated. The results are shown in Table 2.

### (Comparative Example 1)

A photoelectric conversion element was produced in the same manner as in Example 1 except that the cyclic conjugated compound was changed to that described in Table 2 (particle 1 used in Example 1 of Japanese Patent Laid-Open No. 2022-168820), followed by the evaluation of the element. Results are shown in Table 2.

### (Examples 2 to 16, and Comparative Examples 2 and 3)

Photoelectric conversion elements are each produced in the same manner as in Example 1 except that the kind of the cyclic conjugated compound, the presence or absence of the resin solution added in the preparation of a charge-transporting layer solution, the kind of the resin solution added, and the amount of the resin solution added are changed. Results are shown in Table 2.

The cyclic conjugated compounds are each produced in the same manner as in the above-mentioned Step (3) by adjusting the dispersion condition (dispersion time) of an arbitrary cyclic conjugated compound with a sand mill so that a peak ratio may become a ratio shown in the table.

### [Table 2]

**Table 2**

| Example | Charge-transporting layer | | | | | | Second charge-transporting layer | Element characteristic |
|---|---|---|---|---|---|---|---|---|
| | Cyclic conjugated compound | Peak that is present in a range of 5.0 to 8.0° | Peak that is present in a range of 26.0 to 29.0° | Peak ratio | Resin solution | Resin solution addition amount (g) | | Conversion efficiency (%) |
| 1 | Hydroxygallium phthalocyanine | 7.4 | 28.2 | 0.7 | Not used | - | Present | 18.3 |
| 2 | Hydroxygallium phthalocyanine | 7.6 | 28.2 | 1.2 | Not used | - | Present | 18.0 |
| 3 | Hydroxygallium phthalocyanine | 7.6 | 28.3 | 2.0 | Not used | - | Present | 17.8 |
| 4 | Hydroxygallium phthalocyanine | 7.4 | 28.2 | 0.7 | Resin solution 1 | 0.2 | Present | 18.8 |
| 5 | Hydroxygallium phthalocyanine | 7.4 | 28.2 | 0.7 | Resin solution 1 | 0.4 | Present | 17.5 |
| 6 | Hydroxygallium phthalocyanine | 7.4 | 28.2 | 0.7 | Resin solution 1 | 0.1 | Present | 18.0 |
| 7 | Chlorogallium phthalocyanine | 7.4 | 28.3 | 0.7 | Not used | - | Present | 17.3 |
| 8 | Chlorogallium phthalocyanine | 7.3 | 28.1 | 0.7 | Not used | - | Present | 16.8 |
| 9 | Chlorogallium phthalocyanine | 7.4 | 28.3 | 0.8 | Resin solution 1 | 0.2 | Present | 17.5 |
| 10 | Titanyl phthalocyanine | 7.2 | 27.2 | 1.8 | Not used | - | Present | 16.0 |
| 11 | Titanyl phthalocyanine | 7.2 | 27.2 | 0.8 | Not used | - | Present | 15.5 |
| 12 | Titanyl phthalocyanine | 7.2 | 27.2 | 0.9 | Resin solution 2 | 0.2 | Present | 16.5 |
| 13 | Copper phthalocyanine | 6.9 | 27.0 | 1.7 | Not used | - | Present | 15.5 |
| 14 | Phthalocyanine (metal-free) | 7.4 | 28.4 | 0.9 | Not used | - | Present | 15.5 |
| 15 | 2,3,7,8,12,13,17,1 8-Octaphenyl-5,10,15,20-tetraazaporphyrin | 7.9 | 28.6 | 1.9 | Not used | - | Present | 15.3 |
| 16 | 2,3,7,8,12,13,17,1 8-Octaphenyl-5,10,15,20-tetraazaporphyrin | 7.9 | 28.6 | 1.6 | Resin solution 1 | 0.2 | Present | 15.8 |
| Comparative Example 1 | Hydroxygallium phthalocyanine | 7.4 | 28.2 | 2.7 | Not used | - | Present | 15.1 |
| Comparative Example 2 | Titanyl phthalocyanine | 7.2 | 27.2 | 2.5 | Not used | - | Present | 13.2 |
| Comparative Example 3 | 2,3,7,8,12,13,17,1 8-Octaphenyl-5,10,15,20-tetraazaporphyrin | 7.9 | 28.6 | 2.2 | Not used | - | Present | 12.1 |

### (Examples 17 to 19 and Comparative Example 4)

Photoelectric conversion elements are each produced in the same manner as in Example 1 except that: the kind of the cyclic conjugated compound, the presence or absence of the resin solution added in the preparation of a charge-transporting layer solution, the kind of the resin solution added, and the amount of the resin solution added are changed; and the second charge-transporting layer is not formed. Results are shown in Table 3.

The cyclic conjugated compounds are each produced in the same manner as in the above-mentioned Step (3) by adjusting the dispersion condition (dispersion time) of an arbitrary cyclic conjugated compound with a sand mill so that a peak ratio may become a ratio shown in the table.

### [Table 3]

**Table 3**

| Example | Charge-transporting layer | | | | | | Second charge-transporting layer | Element characteristic |
|---|---|---|---|---|---|---|---|---|
| | Cyclic conjugated compound | Peak that is present in a range of 5.0 to 8.0° | Peak that is present in a range of 26.0 to 29.0° | Peak ratio | Resin solution | Resin solution addition amount (g) | | Conversion efficiency (%) |
| 17 | Hydroxygallium phthalocyanine | 7.4 | 28.2 | 0.7 | Not used | - | Absent | 13.3 |
| 18 | Hydroxygallium phthalocyanine | 7.6 | 28.3 | 2.0 | Not used | - | Absent | 12.8 |
| 19 | Titanyl phthalocyanine | 7.2 | 27.2 | 0.8 | Not used | - | Absent | 10.9 |
| Comparative Example 4 | Titanyl phthalocyanine | 7.2 | 27.2 | 2.5 | Not used | - | Absent | 8.2 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, Japanese Patent Application No. 2024-086013 filed on May 28, 2024, and Japanese Patent Application No. 2024-186451 filed on October 23, 2024, and the entire contents thereof are incorporated herein by reference.

### [Reference Signs List]

1 cyclic conjugated compound molecule
2 crystal structure of cyclic conjugated compound according to Examples
3 crystal structure of cyclic conjugated compound according to Comparative Examples
4 substrate
5 second electrode
6 electron-transporting layer
7 photoelectric conversion layer
8 charge-transporting layer
9 first electrode
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element further comprises, between the photoelectric conversion layer and the first electrode, a charge-transporting layer containing a crystal of a cyclic conjugated compound in which a plurality of pyrrole rings are bonded by conjugated bonds,
wherein, in a scanning range of a Bragg angle 2θ of 3.0 to 30.0° in an X-ray diffraction spectrum of the charge-transporting layer using a CuKα ray, when a peak with a maximum intensity out of peaks that are present in a range of 5.0 to 8.0° is defined as a peak α, and a peak with a maximum intensity out of peaks that are present in a range of 26.0 to 29.0° is defined as a peak β, one of the peak α or the peak β is a peak with a maximum intensity in the scanning range, and
wherein, when an intensity of the peak α is defined as Iα and an intensity of the peak β is defined as Iβ, a ratio Iα/Iβ is 2.0 or less.

2. The photoelectric conversion element according to claim 1, wherein the ratio Iα/Iβ is 1.0 or less.

3. The photoelectric conversion element according to claim 1 or 2, further comprising a second charge-transporting layer between the first electrode and the charge-transporting layer.

4. The photoelectric conversion element according to any one of claims 1 to 3, wherein the charge-transporting layer contains a resin.

5. The photoelectric conversion element according to claim 4, wherein a content of the resin in the charge-transporting layer is 3 to 30 mass% with respect to a content of the cyclic conjugated compound.

6. The photoelectric conversion element according to claim 1, wherein the cyclic conjugated compound is a phthalocyanine compound.

7. The photoelectric conversion element according to claim 6, wherein the phthalocyanine compound is a titanyl phthalocyanine compound or a gallium phthalocyanine compound.

8. The photoelectric conversion element according to claim 7, wherein the phthalocyanine compound is a gallium phthalocyanine compound.

9. The photoelectric conversion element according to claim 8, wherein the gallium phthalocyanine compound is a hydroxygallium phthalocyanine compound.

10. The photoelectric conversion element according to any one of claims 1 to 9, wherein the peak α is present in a range of 7.2 to 7.6°, and the peak β is present in a range of 28.0 to 28.4°.

## Amended claims

### Amended claims under Art. 19.1 PCT

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element further comprises, between the photoelectric conversion layer and the first electrode, a charge-transporting layer containing a crystal of a cyclic conjugated compound in which a plurality of pyrrole rings are bonded by conjugated bonds,
wherein, in a scanning range of a Bragg angle 2θ of 3.0 to 30.0° in an X-ray diffraction spectrum of the charge-transporting layer using a CuKα ray, when a peak with a maximum intensity out of peaks that are present in a range of 5.0 to 8.0° is defined as a peak α, and a peak with a maximum intensity out of peaks that are present in a range of 26.0 to 29.0° is defined as a peak β, one of the peak α or the peak β is a peak with a maximum intensity in the scanning range, and
wherein, when an intensity of the peak α is defined as Iα and an intensity of the peak β is defined as Iβ, a ratio Iα/Iβ is 2.0 or less.

2. The photoelectric conversion element according to claim 1, wherein the cyclic conjugated compound is a phthalocyanine compound.

3. The photoelectric conversion element according to claim 11, wherein the phthalocyanine compound is a titanyl phthalocyanine compound or a gallium phthalocyanine compound.

4. The photoelectric conversion element according to claim 11 or 12, wherein the phthalocyanine compound is a gallium phthalocyanine compound.

5. The photoelectric conversion element according to claim 13, wherein the gallium phthalocyanine compound is a hydroxygallium phthalocyanine compound.

6. The photoelectric conversion element according to any one of claims 1 and 11 to 14, wherein the ratio Iα/Iβ is 1.0 or less.

7. The photoelectric conversion element according to any one of claims 1 and 11 to 15, wherein the ratio Iα/Iβ is 0.7 or more.

8. The photoelectric conversion element according to any one of claims 1 and claim 11 to 16, further comprising a second charge-transporting layer between the first electrode and the charge-transporting layer.

9. The photoelectric conversion element according to any one of claims 1 and 11 to 17, wherein the charge-transporting layer contains a resin.

10. The photoelectric conversion element according to claim 18, wherein a content of the resin in the charge-transporting layer is 3 to 30 mass% with respect to a content of the cyclic conjugated compound.

11. The photoelectric conversion element according to any one of claims 1 and 11 to 19, wherein the peak α is present in a range of 7.2 to 7.6°, and the peak β is present in a range of 28.0 to 28.4°.

Statement under Art. 19.1 PCT
Original claims 1 to 10 have been replaced with new claims.

New claim 1 corresponds to the original claim 1.

New claims 11 to 14 correspond to the original claims 6 to 9, respectively.

New claim 15 corresponds to the original claim 2.

New claim 16 had been set a lower limit for Iα/Iβ based on the examples.

New claims 17 to 19 correspond to the original claims 3 to 5, respectively.

New claim 20 corresponds to the original claim 10.
